# EUROPÄISCHE PATENTANMELDUNG

(11) **EP 2 418 198 A1**
(43) Veröffentlichungstag der Anmeldung: **15.02.2012**
(21) Anmeldenummer: 11172650.1
(22) Anmeldetag: 25.07.2007
(51) Int. Cl.: C07C 265/14, C08G 18/10, C09D 175/04

(54) **Pentamethylen-1,5-diisocyanat**

(30) Priorität: 01.08.2006 EP 06118256
(62) Teilanmeldung aus: 07787876.7
(71) Anmelder: BASF SE, 67056 Ludwigshafen (DE)
(72) Erfinder: Fiene, Martin, 67150 Niederkirchen (DE); Stroefer, Eckhard, 68163 Mannheim (DE); Siegel, Wolfgang, 67117 Limburgerhof (DE); Freyer, Stephan, 67434 Neustadt (DE); Zelder, Oskar, 67346 Speyer (DE); Schulz, Gerhard, 67098 Bad Dürkheim (DE)

(57) **Zusammenfassung**

Die vorliegende Erfindung betrifft Pentamethylen-1,5-diisocyanat mit einem ¹⁴C : ¹²C-Isotopenverhältnis von 0,5 × 10⁻¹² bis 5 × 10⁻¹² und dessen Verwendung.

## Beschreibung

Die vorliegende Erfindung betrifft ein Verfahren zur Herstellung von Pentamethylen-1,5-diisocyanat, so hergestelltes Pentamethylen-1,5-diisocyanat und dessen Verwendung.

Die Herstellung von Pentamethylendiisocyanat aus 1,5-Pentandiamin ist an sich bekannt und kann phosgenfrei (T. Lesiak, K. Seyda, Journal für Praktische Chemie (Leipzig), 1979, 321(1), 161 - 163) oder durch Umsetzung mit Phosgen (z.B. DE 2625075) erfolgen.

DE 1900514 (entspr. GB 1225450) beschreibt die zweistufige Herstellung von Pentamethylen-1,5-diisocyanat aus Caprolactam durch Überführung in die Hydroxyamsäuren und deren anschließende Phosgenierung.

Die in dieser Schrift angegebene Ausbeute für die Umwandlung von Caprolactam in Pentamethylen-1,5-diisocyanat beträgt lediglich ca. 32%.

Caprolactam wird großtechnisch entweder mehrstufig aus Benzol durch Kernhydrierung zu Cyclohexan, Oxidation zu Cyclohexanon und Beckmann-Umlagerung mit Hydroxylamin oder aus 1,4-Butadien durch Hydrocyanierung und selektive Hydrierung und anschließende Cyclisierung zum Caprolactam hergestellt. In beiden Fällen ist die Basis ein Kohlenwasserstoff aus der Erdölchemie.

Somit handelt es sich dabei um eine petrochemisch basierte Herstellung über jeweils fünf Stufen ausgehend von Benzol bzw. von Butadien.

Die Herstellung von 1,5-Pentandiamin ist bekannt durch enzymatische Decarboxylierung von Lysin mit beispielsweise Lysin Decarboxylase (EP 1482055 A1 oder JP 2004-222569 A) in einem zellfreien System oder durch thermische oder katalytische Decarboxylierung (G. Gautret de la Moriciere, G. Chatelus, Bull. Soc. Chim. France (1969, 12, 4421 - 4425) oder durch Hydrierung der entsprechenden Nitrile (beispielsweise EP 161419 oder WO 2003/99768).

1,5-Pentandiamin war bisher nicht großtechnisch verfügbar.

WO 2006/005603 beschreibt ein biochemisches Verfahren zur Herstellung von 1,4-Butandiamin aus Ornithin mit Hilfe von Ornithin Decarboxylase und dessen Verwendung als Ausgangsverbindung für die Polyamidherstellung.

Aufgabe der vorliegenden Erfindung war es, Pentamethylen-1,5-diisocyanat herzustellen, das aus nachwachsenden Rohstoffen hergestellt werden kann.

Die Aufgabe wurde gelöst durch ein Verfahren zur Herstellung von Pentamethylen-1,5-diisocyanat, in dem man
b) Lysin in 1,5-Pentandiamin überführt und
c) das so erhaltene 1,5-Pentandiamin in Pentamethylen-1,5-diisocyanat überführt.

Der Vorteil des erfindungsgemäßen Verfahren beruht darin, daß man bei der Herstellung des Pentamethylen-1,5-diisocyanat unabhängig von Erdöl als Rohstoffbasis ist. Zudem weist das auf diese Weise hergestellte Pentamethylen-1,5-diisocyanat eine geringere Farbe auf als konventionell hergestelltes, da es thermisch geringer belastet wird.

Durch die erfindungsgemäße Wahl der Rohstoffbasis Lysin bzw. nachwachsende Rohstoffe erhält man nach dem erfindungsgemäßen Verfahren ein zumindest nahezu isomerenreines Pentamethylen-1,5-diisocyanat, wohingegen das auf herkömmlichem Wege hergestellte Pentamethylen-1,5-diisocyanat einen Anteil an isomeren Pentamethylendiisocyanaten, insbesondere Pentamethylen-1,4-diisocyanat enthält. Dieser Anteil kann abhängig von dessen Herstellung bis zu mehreren Gew% betragen.

Das erfindungsgemäß hergestellte Pentamethylen-1,5-düsocyanat weist dagegen einen Anteil an den verzweigten Pentamethylendiisocyanaten Isomeren von jeweils weniger als 100 ppm auf.

Somit ist ein weiterer Gegenstand des vorliegenden Verfahrens ein Gemisch, bestehend aus mindestens zwei verschiedenen Pentamethylendiisocyanatisomeren, von denen der Hauptbestandteil Pentamethylen-1,5-diisocyanat ist und das in geringeren Mengen enthaltene Isomer in Mengen von nicht mehr als 100 ppm enthalten ist, mit der Maßgabe, daß die Summe 100 Gew% beträgt.

Ein weiterer Gegenstand des vorliegenden Verfahrens ist ein Gemisch, bestehend aus Pentamethylen-1,5-diisocyanat und Pentamethylen-1,4-diisocyanat, wobei der Anteil an Pentamethylen-1,4-diisocyanat nicht mehr als 10000 ppm, bevorzugt 7500 ppm, besonders bevorzugt 5000 ppm, ganz besonders bevorzugt 2500 ppm, insbesondere 1000 ppm, speziell 500 ppm und sogar 100 ppm ausmacht und der Anteil von Pentamethylen-1,5-diisocyanat den Rest zu 100 Gew% ausmacht.

Infolgedessen weist das erfindungsgemäß hergestellte Pentamethylen-1,5-diisocyanat nahezu ausschließlich zwei primäre Isocyanatgruppen auf und zeigt deswegen eine gleichmäßigere Reaktivität in Umsetzungen der Isocyanatgruppen, beispielsweise in der Herstellung von Polyurethanen. Verzweigte Pentamethylendiisocyanat-Isomere dagegen weisen eine primäre und eine sekundäre Isocyanatgruppe auf, die unterschiedlich reaktiv sind.

Das nach dem erfindungsgemäßen Verfahren erhaltene Pentamethylen-1,5-diisocyanat weist in der Regel eine Farbzahl von nicht mehr als 15 APHA gemäß DIN ISO 6271 auf.

Der erfindungsgemäße Schritt b) besteht aus einer Überführung von Lysin in 1,5-Pentandiamin.

Lysin kann in reiner Form eingesetzt werden oder kann im Verlauf der Reaktion erst gebildet werden (siehe unten zu Schritt a)). Weiterhin kann Lysin in Form einer wäßrigen Lösung, Pufferlösung oder als lysinhaltiges Reaktionsgemisch mit einem Lysingehalt von bevorzugt mindestens 5 Gew% bis zur Löslichkeitsgrenze in dem jeweiligen Reaktionsgemisch bei den jeweiligen Temperaturen. In der Regel kann der Gehalt bis zu 45 Gew%, bevorzugt bis zu 40, besonders bevorzugt bis zu 35 und ganz besonders bevorzugt bis zu 30 Gew% betragen.

Das für das erfindungsgemäße Verfahren eingesetzte Lysin (2,6-Diaminohexansäure) stammt aus bevorzugt biologischem Material und kann als D-Enantiomer, als L-Enantiomer, oder als beliebiges Gemisch dieser Enantiomeren, beispielsweise als Racemat, vorliegen, bevorzugt in Form des L-Enantiomers ([(S)-2,6-Diaminohexansäure).

Es kann in freier Form oder als inneres Salz eingesetzt werden, in Form seines Anions als Carboxylat oder einfach oder zweifach protoniert in Form seines Mono- oder Diammoniumsalzes, beispielsweise als Chlorid.

Ferner kann das Lysin in Form seines Esters, beispielsweise als Methyl-, Ethyl, n-Propyl-, iso-Propyl-, n-Butyl-, sek-Butyl- oder iso-Butylesters eingesetzt werden.

Bei dem Schritt b) handelt es sich bevorzugt um eine Decarboxylierung.

In einer Möglichkeit einer Decarboxylierung wird Lysin, gegebenenfalls in einem Lösungsmittel gelöst oder suspendiert, bei einer Temperatur oberhalb von 80 °C, bevorzugt oberhalb von 100 °C, besonders bevorzugt oberhalb von 120 °C, ganz besonders bevorzugt oberhalb von 150 °C und insbesondere oberhalb von 180 °C erhitzt (thermische Decarboxylierung).

Die Temperatur kann bis zu 250 °C betragen, bevorzugt bis zu 230 °C, besonders bevorzugt bis zu 210 °C und ganz besonders bevorzugt bis zu 200 °C.

Gegebenenfalls kann Druck angelegt werden, um eventuell vorhandenes Lösungsmittel im Reaktionsgemisch zu halten.

Beispiele für Lösungsmittel sind aromatische und/oder (cyclo)aliphatische Kohlenwasserstoffe und deren Gemische, halogenierte Kohlenwasserstoffe, Ester, Ether und Alkohole.

Bevorzugt sind aromatische Kohlenwasserstoffe, (cyclo)aliphatische Kohlenwasserstoffe, Alkansäurealkylester, alkoxylierte Alkansäurealkylester und deren Gemische.

Besonders bevorzugt sind ein- oder mehrfach alkylierte Benzole und Naphthaline, Alkansäurealkylester und alkoxylierte Alkansäurealkylester sowie deren Gemische.

Als aromatische Kohlenwasserstoffgemische sind solche bevorzugt, die überwiegend aromatische C₇- bis C₁₄-Kohlenwasserstoffe umfassen und einen Siedebereich von 110 bis 300 °C umfassen können, besonders bevorzugt sind Toluol, o-, m- oder p-Xylol, Trimethylbenzolisomere, Tetramethylbenzolisomere, Ethylbenzol, Cumol, Tetrahydronaphthalin und solche enthaltende Gemische.

Beispiele dafür sind die Solvesso®-Marken der Firma ExxonMobil Chemical, besonders Solvesso® 100 (CAS-Nr. 64742-95-6, überwiegend C₉ und C₁₀-Aromaten, Siedebereich etwa 154 - 178 °C), 150 (Siedebereich etwa 182 - 207 °C) und 200 (CAS-Nr. 64742-94-5), sowie die Shellsol®-Marken der Firma Shell. Kohlenwasserstoffgemische aus Paraffinen, Cycloparaffinen und Aromaten sind auch unter den Bezeichnungen Kristallöl (beispielsweise Kristallöl 30, Siedebereich etwa 158 - 198 °C oder Kristallöl 60: CAS-Nr. 64742-82-1), Testbenzin (beispielsweise ebenfalls CAS-Nr. 64742-82-1) oder Solventnaphtha (leicht: Siedebereich etwa 155 - 180 °C, schwer: Siedebereich etwa 225 - 300 °C,) im Handel erhältlich. Der Aromatengehalt derartiger Kohlenwasserstoffgemische beträgt in der Regel mehr als 90 Gew%, bevorzugt mehr als 95, besonders bevorzugt mehr als 98 und ganz besonders bevorzugt mehr als 99 Gew%. Es kann sinnvoll sein, Kohlenwasserstoffgemische mit einem besonders verringerten Gehalt an Naphthalin einzusetzen.

Halogenierte Kohlenwasserstoffe sind beispielsweise Chlorbenzol und Dichlorbenzol oder dessen Isomerengemische.

Ester sind beispielsweise n-Butylacetat, Ethylacetat, 1-Methoxypropylacetat-2 und 2-Methoxyethylacetat, sowie die Mono- und Diacetylester von Ethylenglykol, Diethylenglykol, Triethylenglykol, Propylenglykol, Dipropylenglykol oder Tripropylenglykol, wie beispielsweise Butylglykolacetat. Weitere Beispiele sind auch Carbonate, wie bevorzugt 1,2-Ethylencarbonat, 1,2-Propylencarbonat oder 1,3-Propylencarbonat.

Ether sind beispielsweise Tetrahydrofuran (THF), Dioxan sowie die Dimethyl-, -ethyl-oder -n-butylether von Ethylenglykol, Diethylenglykol, Triethylenglykol, Propylenglykol, Dipropylenglykol oder Tripropylenglykol.

(Cyclo)aliphatische Kohlenwasserstoffe sind beispielsweise Dekalin, alkyliertes Dekalin und Isomerengemische von geradlinigen oder verzweigten Alkanen und/oder Cycloalkanen.

Alkohole sind beispielsweise Methanol, Ethanol, n-Propanol, iso-Propanol, n-Butanol, sek-Butanol, iso-Butanol, Pentanol-Isomerengemische, Hexanol-Isomerengemische, 2-Ethylhexanol oder Oktanol.

### Besonders geeignet ist Wasser.

Zur Decarboxylierung kann zusätzlich noch eine Base zugegeben werden, beispielsweise eine organische Base, bevorzugt ein Amin, besonders bevorzugt ein sekundäres oder tertiäres Amin, oder eine anorganische Base, wie beispielsweise Alkali- oder Erdalkalimetalloxide, -hydroxide, -carbonate oder -hydrogencarbonate, bevorzugt Natronlauge, Kalilauge, Natriumhydrogencarbonat, Natriumcarbonat, Kaliumhydrogencarbonat, Kalziumhydroxid, Kalkmilch oder Kaliumcarbonat (katalytische Decarboxylierung).

Insbesondere wenn Lysin in Form eines Esters, bevorzugt des Methylesters eingesetzt wird, ist eine Durchführung der Reaktion als Desalkoxycarbonylierung unter sogenannten "Krapcho"-Bedingungen bevorzugt, wobei dem Reaktionsgemisch ein Nukleophil, bevorzugt eine Iodid oder Bromid, besonders bevorzugt ein Iodid zugegeben wird und unter diesen Reaktionsbedingungen erhitzt wird.

Besonders bevorzugt wird jedoch die Decarboxylierung mit Hilfe eines Enyzyms durchgeführt,

Bevorzugt handelt es sich um Lyasen (E.C. 4.-.-.-), besonders bevorzugt um Kohlenstoff-Kohlenstoff-Lyasen (E.C. 4.1.-.-) und ganz besonders bevorzugt um Carboxy-Lyasen (E.C. 4.1.1.-)

Beispiele dafür sind:
EC 4.1.1.1 pyruvate decarboxylase
EC 4.1.1.2 oxalate decarboxylase
EC 4.1.1.3 oxaloacetate decarboxylase
EC 4.1.1.4 acetoacetate decarboxylase
EC 4.1.1.5 acetolactate decarboxylase
EC 4.1.1.6 aconitate decarboxylase
EC 4.1.1.7 benzoylformate decarboxylase
EC 4.1.1.8 oxalyl-CoA decarboxylase
EC 4.1.1.9 malonyl-CoA decarboxylase
EC 4.1.1.11 aspartate 1-decarboxylase
EC 4.1.1.12 aspartate 4-decarboxylase
EC 4.1.1.14 valine decarboxylase
EC 4.1.1.15 glutamate decarboxylase
EC 4.1.1.16 hydroxyglutamate decarboxylase
EC 4.1.1.17 ornithine decarboxylase
EC 4.1.1.18 lysine decarboxylase
EC 4.1.1.19 arginine decarboxylase
EC 4.1.1.20 diaminopimelate decarboxylase
EC 4.1.1.21 phosphoribosylaminoimidazole carboxylase
EC 4.1.1.21 phosphoribosylaminoimidazole carboxylase
EC 4.1.1.22 histidine decarboxylase
EC 4.1.1.23 orotidine-5'-phosphate decarboxylase
EC 4.1.1.24 aminobenzoate decarboxylase
EC 4.1.1.25 tyrosine decarboxylase
EC 4.1.1.28 aromatic-L-amino-acid decarboxylase
EC 4.1.1.29 sulfoalanine decarboxylase
EC 4.1.1.30 pantothenoylcysteine decarboxylase
EC 4.1.1.31 phosphoenolpyruvate carboxylase
EC 4.1.1.32 phosphoenolpyruvate carboxykinase (GTP)
EC 4.1.1.33 diphosphomevalonate decarboxylase
EC 4.1.1.34 dehydro-L-gulonate decarboxylase
EC 4.1.1.35 UDP-glucuronate decarboxylase
EC 4.1.1.36 phosphopantothenoylcysteine decarboxylase
EC 4.1.1.37 uroporphyrinogen decarboxylase
EC 4.1.1.38 phosphoenolpyruvate carboxykinase (diphosphate)
EC 4.1.1.39 ribulose-bisphosphate carboxylase
EC 4.1.1.40 hydroxypyruvate decarboxylase
EC 4.1.1.41 methylmalonyl-CoA decarboxylase
EC 4.1.1.42 carnitine decarboxylase
EC 4.1.1.43 phenylpyruvate decarboxylase
EC 4.1.1.44 4-carboxymuconolactone decarboxylase
EC 4.1.1.45 aminocarboxymuconate-semialdehyde decarboxylase
EC 4.1.1.46 o-pyrocatechuate decarboxylase
EC 4.1.1.47 tartronate-semialdehyde synthase
EC 4.1.1.48 indole-3-glycerol-phosphate synthase
EC 4.1.1.49 phosphoenolpyruvate carboxykinase (ATP)
EC 4.1.1.50 adenosylmethionine decarboxylase
EC 4.1.1.51 3-hydroxy-2-methylpyridine-4,5-dicarboxylate 4-decarboxylase
EC 4.1.1.52 6-methylsalicylate decarboxylase
EC 4.1.1.53 phenylalanine decarboxylase
EC 4.1.1.54 dihydroxyfumarate decarboxylase
EC 4.1.1.55 4,5-dihydroxyphthalate decarboxylase
EC 4.1.1.56 3-oxolaurate decarboxylase
EC 4.1.1.57 methionine decarboxylase
EC 4.1.1.58 orsellinate decarboxylase
EC 4.1.1.59 gallate decarboxylase
EC 4.1.1.60 stipitatonate decarboxylase
EC 4.1.1.61 4-hydroxybenzoate decarboxylase
EC 4.1.1.62 gentisate decarboxylase
EC 4.1.1.63 protocatechuate decarboxylase
EC 4.1.1.64 2,2-dialkylglycine decarboxylase (pyruvate)
EC 4.1.1.65 phosphatidylserine decarboxylase
EC 4.1.1.66 uracil-5-carboxylate decarboxylase
EC 4.1.1.67 UDP-galacturonate decarboxylase
EC 4.1.1.68 5-oxopent-3-ene-1,2,5-tricarboxylate decarboxylase
EC 4.1.1.69 3,4-dihydroxyphthalate decarboxylase
EC 4.1.1.70 glutaconyl-CoA decarboxylase
EC 4.1.1.71 2-oxoglutarate decarboxylase
EC 4.1.1.72 branched-chain-2-oxoacid decarboxylase
EC 4.1.1.73 tartrate decarboxylase
EC 4.1.1.74 indolepyruvate decarboxylase
EC 4.1.1.75 5-guanidino-2-oxopentanoate decarboxylase
EC 4.1.1.76 arylmalonate decarboxylase
EC 4.1.1.77 4-oxalocrotonate decarboxylase
EC 4.1.1.78 acetylenedicarboxylate decarboxylase
EC 4.1.1.79 sulfopyruvate decarboxylase
EC 4.1.1.80 4-hydroxyphenylpyruvate decarboxylase
EC 4.1.1.81 threonine-phosphate decarboxylase
EC 4.1.1.82 phosphonopyruvate decarboxylase
EC 4.1.1.83 4-hydroxyphenylacetate decarboxylase
EC 4.1.1.84 D-dopachrome decarboxylase
EC 4.1.1.85 3-dehydro-L-gulonate-6-phosphate decarboxylase

Insbesondere bevorzugt ist die (enzymatische Decarboxylierung) in Gegenwart von Lysin-Decarboxylase (E.C. 4.1.1.18, besonders CAS-Nr. 9024-76-4).

Dabei ist es zunächst nicht erforderlich, zwischen der Durchführung des Schrittes b) in einem zellfreien System und einer fermentativen Durchführung zu unterscheiden. Eine besonders bevorzugte Ausführungsform der vorliegenden Erfindung besteht jedoch darin, das 1,5-Pentandiamin fermentativ durch lebende Mikroorganismen aus geeigneten Substraten herzustellen.

Besonders bevorzugt wird die Decarboxylierung in Gegenwart genetisch veränderter Mikroorganismen durchgeführt, wie beispielsweise beschrieben in der EP 1482055 und in der Internationalen Patentanmeldung mit dem Aktenzeichen PCT/EP2007/052783, dem Einreichungsdatum 23. März 2007 und dem Titel "Process for the production of cadaverine", die beide hiermit durch Referenznahme Bestandteil dieser Offenbarung seien.

Bevorzugte Mikroorganismen sind genetisch veränderte rekombinante Mikroorganismen, die Gene mit Lysin-Decarboxylase-Aktivität tragen, bevorzugt das cadA Gen (Kyoto Encyclopedia of Genes and Genomes, Entry b4131) und das IdcC Gen (Kyoto Encyclopedia of Genes and Genomes, Entry JW0181) von Escherichia coli.

Besonders bevorzugt handelt es sich bei den Mikroorganismen um Corynebacterien und besonders bevorzugt um Corynebacterium glutamicum.

Es stellt eine besonders bevorzugte Ausführungsform der vorliegenden Erfindung dar, anstelle des Schrittes b) (Umwandlung von Lysin in 1,5-Pentandiamin) eine einstufige Synthese von 1,5-Pentandiamin ausgehend von einem geeigneten Substrat in einem Schritt a) durchzuführen. Dies umfaßt den Schritt b) in der Regel in Form einer intrazellulären Umwandlung des Substrats in Lysin und anschließend die ebenfalls intrazelluläre Umwandlung von Lysin in 1,5-Pentandiamin.

Dabei spielt es erfindungsgemäß keine Rolle, ob Lysin in reiner Form isoliert wird, in einem als Zwischenprodukt erhaltenen Gemisch enthalten ist oder im Verlauf des Schrittes a) lediglich intermediär, beispielsweise intrazellulär gebildet wird. Bei letzterer Variante spielt es darüberhinaus keine Rolle, ob tatsächlich Lysin als Intermediat entsteht oder ob das Intermediat lediglich eine Lysin-Grundstruktur aufweist und beispielsweise die Carboxylgruppe verestert oder die Aminogruppen substituiert sind.

Ohne an eine Theorie gebunden sein zu wollen wird vermutet, daß in der Biosynthese von Lysin ein Monosaccharid über eine Anzahl von Zwischenstufen in Asparaginsäure umgewandelt wird, das nach Umwandlung in 4-Oxo-2-Aminobuttersäure mit Pyruvat zu dem Intermediat Dihydropicolin-2,6-dicarbonsäure reagiert. Diese wird in Tetrahydropicolin-2,6-dicarbonsäure umgewandelt, die schließlich zu Diaminopimelinsäure reagiert, das durch Decarboxylierung in Lysin umgewandelt wird.

Ein bevorzugtes Verfahren zur Durchführung des Schrittes a) ist beschrieben in der Internationalen Patentanmeldung mit dem Aktenzeichen PCT/EP2007/052783, dem

Einreichungsdatum 23. März 2007 und dem Titel "Process for the production of cadaverine", die hiermit durch Referenznahme Bestandteil dieser Offenbarung sei.

Geeignete Substrate für die Reaktion sind nachwachsende Rohstoffe. Dabei handelt es sich gemäß der Definition von Römpp-Online, Stichwort "Nachwachsende Rohstoffe", Dokument RD-14-00046, Stand August 2005, um landwirtschaftlich und forstwirtschaftlich erzeugte Produkte, die einer Verwendung im Nichtnahrungsbereich zugeführt werden. Demnach zählen zu den nachwachsenden Rohstoffen sowohl primäre Rohstoffe, wie Holz, als auch Produkte der ersten und zweiten Verarbeitungsstufe wie Cellulose, Stärke, monomere Kohlenhydrate, Chitin, tierische oder pflanzliche Fette und Öle, sowie Proteine und tierische Produkte, wie z.B. Schurwolle, Leder und Häute, Talg, Gelatine und Casein, sowie organische Rückstände, wie Stroh. Bei Stärke kann es sich beispielsweise um solche von Kartoffeln, Cassava, Getreide, z.B. Weizen, Mais, Gerste, Roggen, Triticale oder Reis, und verschiedenen Hirsesorten, z.B. Sorghum und Milo, handeln.

Bevorzugte geeignete Substrate sind Monosaccharide, Oligosaccharide und Polysaccharide von Pentosen und/oder Hexosen, wie beispielsweise Mannose, Galactose, Sorbose, Xylose, Arabinose, Ribose, Glucose, Sucrose, Lactose, Fructose, Maltose, Melasse, Stärke oder Cellulose, aber auch Öle und Fette, wie z.B. Sojaöl, Sonnenblumenöl, Erdnußöl, Kokusnußöl oder Rapsöl, oder Fettsäuren, wie z.B. Palmitinsäure, Stearinsäure und Linolensäure, oder Alkohole, wie Glycrin und Ethanol, oder organische Säuren, wie z.B. Essigsäure. In einer bevorzugten Ausführungsform werden Glucose, Fructose oder Sucrose als Kohlenstoffquelle eingesetzt. Diese Verbindungen können einzeln oder als Gemisch eingesetzt werden.

Ein bevorzugtes Verfahren zur Umwandlung von Stärke in Lysin ist beispielsweise beschrieben in WO 05/116228, die hiermit durch Referenznahme Bestandteil dieser Offenbarung sei.

Als Stickstoffquellen können organische Verbindungen eingesetzt werden, die Stickstoff etnhalten, beispielsweise Pepton, Hefeextrakt, Fleischextrakt, Malzextrakt, Sojamehl und Harnstoff, oder anorganische Verbindungen, wie Ammonium sulfat, Ammonium chlorid, Ammonium phosphat, Ammonium carbonat und Ammonium nitrat oder ein Gemisch der genannten Verbindungen eingesetzt werden.

Phosphorquellen, die eingesetzt werden können, sind Phosphorsäure, Kaliumdihydrogenphosphat, Dikaliumhydrogenphosphat, oder die entsprechenden Natriumverbindungen.

Das Kulturmedium kann weiterhin Metallsalze enthalten, beispielsweise Magnesium sulfate oder Eisen sulfat, die zum Wachstum nötig sind.

Weiterin können essentielle wachstumsfördernde Verbindungen, wie z.B. Aminosäuren oder Vitamine zusätzlich zu den oben genannten Verbindungen eingesetzt werden. Entsprechende Vorläufer können ebenfalls zum Kulturmedium zugegeben werden.

Die enzymatische Decarboxylierung erfolgt im Allgemeinen bei 0 bis 100°C, bevorzugt 20 bis 80 °C, besonders bevorzugt 20 bis 70°C, ganz besonders bevorzugt 20 bis 60 °C.

Der pH-Wert des Kulturmediums wird in der Regel zwischen 6,0 und 8,5 gehalten.

Der Enzymgehalt im Reaktionsmedium liegt in der Regel im Bereich von etwa 0,1 bis 10 Gew.-%, bezogen auf das eingesetzte Lysin.

Die Reaktionszeit hängt unter anderem von der Temperatur, der verwendeten Menge und der Aktivität des Enzymkatalysators oder des Mikroorganismus und vom geforderten Umsatz ab. Bevorzugt wird die Reaktionszeit so angepaßt, daß der Umsatz aller ursprünglich im Lysin enthaltenden Carboxyfunktionen mindestens 70%, bevorzugt mindestens 80, besonders bevorzugt mindestens 90, ganz besonders bevorzugt mindestens 95 %, insbesondere mindestens 98% und speziell mindestens 99% beträgt. In der Regel sind dafür 1 bis 48 Stunden und bevorzugt 1 bis 12 Stunden ausreichend.

Es kann erforderlich sein, Sauerstoff durch das Reaktionsgemisch zu leiten.

Die Reaktion kann in organischen Lösungsmitteln oder deren Gemischen oder ohne Zusatz von Lösungsmitteln ablaufen. Als Lösungsmittel kann aber auch Wasser eingesetzt werden.

Der Anteil organischer Lösungsmittel beträgt beispielsweise 0,01-90 Gew.-%. Geeignete organische Lösungsmittel sind solche für diese Zwecke bekannten, beispielsweise tertiäre Monoole, wie C₃-C₆-Alkohole, bevorzugt tert-Butanol, tert-Amylalkohol, Pyridin, Poly-C₁-C₄-alkylenglykoldi-C₁-C₄-alkylether, bevorzugt Polyethylenglycoldi-C₁-C₄-alkylether, wie z.B. 1,2-Dimethoxyethan, Diethylenglycoldimethylether, Polyethylenglycoldimethylether 500, C₁-C₄-Alkylencarbonate, insbesondere Propylencarbonat, C₃-C₆-Alkylessigsäureester, insbesondere tert.-Butyl-essigsäureester, THF, Toluol, 1,3-Dioxolan, Aceton, iso-Butyl-methylketon, Ethylmethylketon, 1,4-Dioxan, tert-Butylmethylether, Cyclohexan, Methylcyclohexan, Toluol, Hexan, Dimethoxymethan, 1,1-Dimethoxyethan, Acetonitril, sowie deren ein- oder mehrphasige Mischungen.

Wahlweise können zu den organischen Lösungsmitteln wässrige Lösungsmittel zugesetzt werden, so dass - je nach organischem Lösungsmittel - ein- oder mehrphasige Reaktionslösungen entstehen. Beispiele für wässrige Lösungsmittel sind Wasser sowie wässrige, verdünnte (z.B. 10 bis 100mM) Puffer, beispielsweise mit einem pH-Wert im Bereich von etwa 6 bis 8, wie z.B. Kaliumphosphat- oder TRIS-HCI-Puffer.

Die Substrate liegen entweder gelöst, als Feststoffe suspendiert oder in Emulsion im Reaktionsmedium vor. Vorzugsweise liegt die anfängliche Konzentration der Reaktanden im Bereich von etwa 0,1 bis 20 Mol/l, insbesondere bei 0,15 bis 10 Mol/l oder 0,2 bis 5 mol/l liegt.

Die Reaktion kann kontinuierlich, beispielsweise in einem Rohrreaktor oder in einer Rührreaktorkaskade, oder diskontinuierlich erfolgen.

Die Umsetzung kann in allen für eine solche Umsetzung geeigneten Reaktoren durchgeführt werden. Solche Reaktoren sind dem Fachmann bekannt. Bevorzugt erfolgt die Umsetzung in einem Rührkesselreaktor oder einem Festbettreaktor.

Zur Durchmischung des Reaktionsansatzes können beliebige Verfahren eingesetzt werden. Spezielle Rührvorrichtungen sind nicht erforderlich. Das Reaktionsmedium kann ein- oder mehrphasig sein und die Reaktanden werden darin gelöst, suspendiert oder emulgiert, gegebenenfalls vorgelegt und zum Start der Reaktion, sowie gegebenenfalls ein- oder mehrmals im Verlauf der Reaktion, mit dem Enzympräparat versetzt. Die Temperatur wird während der Reaktion auf den gewünschten Wert eingestellt und kann, falls gewünscht, während des Reaktionsverlauf erhöht oder verringert werden.

Wird die Reaktion im Festbettreaktor durchgeführt, so ist der Festbettreaktor bevorzugt mit immobilisierten Enzymen bestückt, wobei die Reaktionsmischung durch eine mit dem Enzym gefüllte Säule gepumpt wird. Es ist auch möglich, die Umsetzung im Wirbelbett durchzuführen, wobei das Enzym auf einem Träger immobilisiert eingesetzt wird. Die Reaktionsmischung kann kontinuierlich durch die Säule gepumpt werden, wobei mit der Fließgeschwindigkeit die Verweilzeit und damit der gewünschte Umsatz steuerbar ist. Es ist auch möglich, die Reaktionsmischung im Kreislauf durch eine Säule zu pumpen.

Nach Beendigung der Reaktion kann man das aus b) bzw. a) erhältliche Reaktionsgemisch ohne weitere Aufreinigung weiterverwenden oder es bevorzugt aufreinigen, bevor man es in den Schritt c) einsetzt.

Das aus dem vorhergehenden Reaktionsschritt erhaltene Reaktionsgemisch enthält in der Regel neben 1,5-Pentandiamin und Wasser noch unumgesetztes Substrat, Metaboliten des eingesetzten Subtsrats sowie gegebenenfalls organische Lösungsmittel, und weiterhin eventuell Enzym, intakte oder lysierte Mikroorganismen.

In der Regel wird lediglich das eingesetzte Enzym sowie vom Reaktionsgemisch abgetrennt und das Reaktionsprodukt vom gegebenenfalls verwendeten organischen Lösungsmittel abgetrennt.

Eine Abtrennung vom Enzym erfolgt in der Regel durch Kristallisation, Fällung, Chromatographie, Reversosmose, Elektrophorese, Elektrodialyse, Extraktion, Destillation, Filtration, Absorption, Zentrifugation oder Dekantieren. Das abgetrennte Enzym kann anschließend für weitere Reaktionen eingesetzt werden.

Eine Abtrennung vom Mikroorganismen oder Lysat erfolgt in der Regel durch Extraktion, Destillation, Filtration, Absorption, diskontinuierliche oder kontinuierliche Zentrifugation, Querstromzentrifugation oder Dekantieren. Abgetrennte intakte Mikroorganismen können anschließend für weitere Reaktionen eingesetzt werden.

Vor der Abtrennung können Mikroorganismen, falls erwünscht, noch aufgeschlossen werden, z.B. durch Scherung.

Die Abtrennung vom organischen Lösungsmittel erfolgt in der Regel durch Destillation, Rektifikation.

Zur Destillation kann eine Destillationskolonne mit 1 bis 20 theoretischen Böden auf das Reaktionsgefäß aufgesetzt werden, in der der Rücklauf den Trennerfordernissen angepaßt werden kann. Bei niedrigsiedenden organischen Lösungsmittel kann auch eine einstufige Destillation über Flash-, Fallfilm-, Dünnfilm-, Kurzweg- und/oder Wischblattverdampfer, denen gegebenenfalls eine kurze Kolonne aufgesetzt sein kann.

Die Abtrennung der Leichtsieder aus dem Reaktionsgemisch kann durch das Durchleiten eines unter den Reaktionsbedingungen im wesentlichen inerten Gasstromes (Strippen), wie z.B. ein sauerstoffabgereichertes Gemisch aus Luft und Stickstoff (Magerluft) oder bevorzugt Stickstoff oder Kohlenstoffdioxid unterstützt werden.

Die Entfernung des Wassers erfolgt dann bevorzugt kontinuierlich oder schrittweise in an sich bekannter Weise, z.B. durch Vakuum, azeotrope Entfernung, Absorption, Pervaporation und Diffusion über Membranen.

Es kann auch möglich sein, 1,5-Pentandiamin in ein Salz, bevorzugt in das Hydrochlorid zu überführen und mit wasserlöslichen organischen Lösungsmittel, beispielsweise Alkoholen oder Aceton, auszufällen. In diesem Fall kann man das Präzipitat durch Wäsche und/oder Kristallisation aufreinigen und das 1,5-Diamin anschließend durch Zugabe einer Base wieder freisetzen.

Zur Absorption eignen sich vorzugsweise Molekularsiebe oder Zeolithe (Porengröße z.B. im Bereich von etwa 3-10 Angström), alternativ eine Abtrennung durch Destillation oder mit Hilfe geeigneter semipermeabler Membranen.

Das so erhaltene 1,5-Pentandiamin kann, falls erforderlich, nochmals destilliert werden, so daß der Reinheitsgrad in der Regel bei mindestens 98%, bevorzugt mindestens 99% besonders bevorzugt mindestens 99,5% und ganz besonders bevorzugt mindestens 99,8% liegt.

Der Schritt c) kann phosgenfrei oder in Gegenwart von Phosgen erfolgen, bei letzterer Variante kann die Phosgenierung in Flüssigphase oder in Gasphase erfolgen.

Phosgenfreie Verfahren zur Herstellung von Isocyanaten sind bekannt beispielsweise aus der EP 18588 A1, EP 28338 A2, EP 27952, EP 126299 und besonders EP 566925 A2.

Die im Stand der Technik bekannten phosgenfreien Verfahren können für das erfindungsgemäße Verfahren angewendet werden, bevorzugt jedoch das im folgenden beschriebene:

Zur Herstellung der Urethane wird das Amin mit Harnstoff und mindestens einem, bevorzugt genau einem Alkohol in einem molaren Verhältnis von Amin, Harnstoff und Alkohol wie 1 : 2 bis 20 : 5 bis 40 bei Temperaturen von 50 - 300 °C und insbesondere bei 180 - 220 °C unter einem Druck von 0,1 bis 30 bar, vorzugsweise 5 - 20 bar zur Reaktion gebracht. Bei diesen Reaktionsbedingungen ergeben sich für das Verfahren mittlere Reaktionszeiten von Bruchteilen von Sekunden bis Minuten.

Die Umsetzung kann in Gegenwart von Dialkylcarbonaten, zweckmäßigerweise in einer Menge von 0,1 bis 30 Mol%, vorzugsweise 1 bis 10 Mol% oder Carbamidsäurealkylestern zweckmäßigerweise in einer Menge von 1 bis 20 Mol%, vorzugsweise von 5 bis 15 Mol%, bezogen auf das Diamin, durchgeführt werden. Insbesondere verwendet werden dabei Mischungen aus Dialkylcarbonaten und Carbamidsäurealkylestern in den genannten Mengenverhältnissen. Als Dialkylcarbonate und/oder Carbamidsäureester setzt man bevorzugt solche ein, deren Alkylreste dem Alkylrest des verwendeten Alkohols entsprechen.

Die Umsetzung kann auch in Gegenwart von Katalysatoren erfolgen. Diese werden zweckmäßigerweise in Mengen von 0,001 bis 20 Gew% vorzugsweise 0,001 bis 5 Gew% insbesondere 0,01 bis 0,1 Gew%, bezogen auf das Gewicht des Amins, eingesetzt.

Als Katalysatoren eignen sich anorganische oder organische Verbindungen, die ein oder mehrere Kationen, vorzugsweise ein Kation von Metallen der Gruppe IA, IB, IIA, IIB, IIIB, IVA, IVB, VA, VB, VIB, VIIB, VIIIB des Periodensystems der Elemente enthalten, definiert gemäß Handbook of Chemistry and Physics 14th Edition, publiziert von Chemical Rubber Publishing Co., 23 Superior Ave. N.E., Cleveland, Ohio, enthalten. Beispielhaft genannt seien die Kationen folgender Metalle: Lithium, Natrium, Kalium, Magnesium, Calcium, Aluminium, Gallium, Zinn, Blei, Bismut, Antimon, Kupfer, Silber, Gold, Zink, Quecksilber, Cer, Titan, Vanadium, Chrom, Molybdän, Mangan, Eisen und Cobalt.

Der Katalysator kann weiterhin mindestens ein Anion enthalten, beispielsweise Halogenide, wie Chloride und Bromide, Sulfate, Phosphate, Nitrate, Borate, Alkoholate, Phenolate, Sulfonate, Oxide, Oxidhydrate, Hydroxide, Carboxylate, Chelate, Carbonate und Thio- oder Dithiocarbamate.

Die Katalysatoren können ohne erkennbare deutliche Nachteile auch in Form ihrer Hydrate oder Ammoniakate zum Einsatz kommen.

Als typische Katalysatoren seien beispielhaft folgende Verbindungen genannt: Lithiummethanolat, Lithiumethanolat, Lithiumpropanolat, Lithiumbutanolat, Natriummethanolat, Kalium-tert.-butanolat, Magnesiummethanolat, Calciummethanolat, Zinn-(II)-chlorid, Zinn-(IV)-chlorid, Bleiacetat, Bleiphosphat, Antimon-(III)-chlorid, Antimon-(V)-chlorid, Aluminiumacetylacetonat, Aluminium-iso-butylat, Aluminiumtrichlorid, Bismut-(III)-chlorid, Kupfer-(II)-acetat, Kupfer-(II)-sulfat, Kupfer-(II)-nitrat, Bis-(triphenylphosphinoxido)-kupfer-(II)-chlorid, Kupfermolybdat, Silberacetat, Goldacetat, Zinkoxid, Zinkchlorid, Zinkacetat, Zinkacetonylacetat, Zinkoctoat, Zinkoxalat, Zinkhexylat, Zinkbenzoat, Zinkundecylenat, Cer-(IV)-oxid, Uranylacetat, Titantetrabutanolat, Titantetrachlorid, Titantetraphenolat, Titannaphtenat, Vanadium-(III)-chlorid, Vanadiumacetylacetonat, Chrom-(III)-chlorid, Molybdän-(VI)-oxid, Molybdänacetylacetonat, Wolfram-(VI)-oxid, Mangan-(II)-chlorid, Mangan-(II)-acetat, Mangan-(III)-acetat, Eisen-(II)-acetat, Eisen-(III)-acetat, Eisenphosphat, Eisenoxalat, Eisen-(III)-chlorid, Eisen-(III)-bromid, Cobaltacetat, Cobaltchlorid, Cobaltsulfat, Cobaltnaphthenat, Nickelchlorid, Nickelacetat und Nickelnaphthenat sowie deren Gemische.

Als bevorzugte Katalysatoren seien beispielhaft folgende Verbindungen genannt: Lithiumbutanolat, Aluminiumacetylacetonat, Zinkacetylacetonat, Titantetrabutanolat und Zirkontetrabutylat.

Die Vermischung der Eduktströme kann bevorzugt in einer geeigneten speziellen Mischeinrichtung erfolgen, die sich durch geringe Mischzeiten auszeichnet.

Der vermischte Eduktstrom wird dann in eine Reaktionseinrichtung geführt, die rückvermischt oder als Rohrreaktor oder als Kombination daraus gestaltet sein kann.

Das Reaktionsgemisch wird im Reaktor bei einer mittleren von 10 Sekunden bis 5 Stunden, bevorzugt 20 Sekunden bis 20 Minuten, besonders bevorzugt 30 Sekunden bis 10 Minuten umgesetzt. Die Temperatur beträgt im allgemeinen zwischen 50 °C und 300 °C, bevorzugt zwischen 180 °C und 220 °C. Der Druck beträgt in der Regel zwischen 0,1 bar abs und 30 bar abs und bevorzugt zwischen 5 und 20 bar abs.

Die Verweilzeit ist so gewählt, daß der Umsatz, bezogen auf Aminogruppen im eingesetzten Amin zu Urethangruppen, nach Verlassen des Reaktors mindestens 95%, bevorzugt mindestens 98, besonders bevorzugt mindestens 99 und ganz besonders bevorzugt mindestens 99,5% beträgt.

Ist der Umsatz, bezogen auf Aminogruppen im eingesetzten Amin zu Urethangruppen, nach Verlassen des Reaktors noch nicht vollständig und beträgt beispielsweise weniger als 95%, so kann der Austrag nochmals nachreagiert werden.

Zur Abtrennung des Ammoniaks werden zweckmäßigerweise Kolonnen verwendet, bevorzugt wird der Ammonik per Destillation abgetrennt. Dadurch gelingt eine gute Trennung zwischen dem Alkohol und Ammoniak. Üblicherweise erfolgt die Abtrennung in einem Druckbereich von 0,01 - 20 bar, vorzugsweise bei 0,04 - 15 bar. Die notwendigen Temperaturen richten sich nach dem verwendeten Alkohol bzw. dem Alkoholgemisch. Für n-Butanol liegt die Temperatur beispielsweise bei 60 - 150 °C, bevorzugt bei 80 bis 140 °C.

Es hat sich als vorteilhaft erwiesen, das entstehende Ammoniak sofort aus der Reaktionsmischung abzutrennen, so daß eine Belegung durch Ammoniumcarbaminat, welches in minimalen Mengen aus Ammoniak und Kohlendioxid durch Zersetzung von Harnstoff gebildet wird, vermieden werden kann.

Diese Destillationseinheit ist von an sich bekannter Bauart und weist die üblichen Einbauten auf. Als Kolonneneinbauten kommen prinzipiell alle gängigen Einbauten in Betracht, beispielsweise Böden, Packungen und/oder Schüttungen. Von den Böden sind Glockenböden, Siebböden, Ventilböden, Thormannböden und/oder Dual-Flow-Böden bevorzugt, von den Schüttungen sind solche mit Ringen, Wendeln, Sattelkörpern, Raschig-, Intos- oder Pall-Ringen, Barrel- oder Intalox-Sätteln, Top-Pak etc. oder Geflechten bevorzugt. Bevorzugt werden Böden verwendet, besonders bevorzugt Glockenböden.

Die Destillationskolonne weist bevorzugt 10 - 20 theoretische Trennböden auf.

Aus der erhaltenen ammoniakabgereicherten Reaktionsmischung werden dann Alkohol, Dialkylcarbonate, sofern solche gebildet wurden oder in der Reaktionsmischung vorliegen, oder Carbamidsäurealkylester oder Mischungen aus mindestens zwei dieser Komponenten abgetrennt und vorzugsweise in die Reaktionsstufe zurückgeführt.

Zur Abtrennung der Komponenten wird die Reaktionsmischung vorteilhafterweise vom Druckniveau der Reaktionsstufe auf einen Druck im Bereich von 1 bis 500 mbar, vorzugsweise von 10 bis 100 mbar entspannt. Man erhält hierbei gasförmige Brüden, die die überwiegende Alkoholmenge sowie 0 bis 30 Gew.%, vorzugsweise 1 bis 10 Gew.% Dialkylcarbonat und/oder 1 bis 50 Gew.%, vorzugsweise 1 bis 20 Gew.% Carbamidsäurealkylester enthalten, und einen flüssigen Austrag, der im wesentlichen aus dem monomeren Diurethan besteht und gegebenenfalls Oligoharnstoff-polyurethanen und hochsiedende Oligomere enthält.

Die erhaltenen Brüden werden in nachfolgenden zweckmäßigerweise destillativen Reinigungsstufen, vorzugsweise durch Rektifikation, getrennt und die hierbei isolierten Wertprodukte Alkohol und Carbamidsäurealkylester, einzeln oder als Mischung, vorzugsweise in die Reaktionsstufe zur Bildung der monomeren Urethane zurückgeführt.

Zur destillativen Abtrennung des Alkohols oder des Alkoholgemisches wird häufig ein sogenannter Flash eingesetzt. Dieser Apparat kann ein Behälter oder eine Kombination von Behälter und Kolonne vorzugsweise eine Kolonne sein, wobei im Kopf der Alkohol bzw. das Alkoholgemisch und im Sumpf das Urethan abgezogen werden kann. Im Kopf der Kolonne können neben dem Alkohol auch weitere leichter als das Urethan siedende Stoffe enthalten sein. Die Trennung erfolgt in einem Druckbereich von 0,001 bis 1 bar, vorzugsweise bei 0,02 - 0,5 bar.

Die nach Abtrennung der Brüden in der Regel als Sumpfaustrag erhaltene flüssige, die monomeren Diurethane, und gegebenenfalls Oligoharnstoff-polyurethane und hochsiedende Oligomere enthaltende Reaktionsmischung kann entweder vollständig in die Folgestufe geführt werden oder wird bevorzugt in zwei Teilströme geteilt, wobei das Gewichtsverhältnis der Teilmengen 5 bis 50:95 bis 50 Gew.-Teile, vorzugsweise 10 bis 30:90 bis 70 Gew.-Teile beträgt.

Die gleich große oder vorzugsweise kleinere Teilmenge wird destillativ getrennt mittels einer üblichen Destillationsanlage, vorzugsweise eines Dünnschichtverdampfers, bei einer Temperatur von 170 bis 240°C, vorzugsweise von 180 bis 230°C und unter einem Druck von 0,001 - 1 bar, vorzugsweise 0,002 - 0,01 bar, in ein Wertprodukt, das die Diurethane und die leichter siedende Nebenprodukte enthält, und nicht destillierbare Nebenprodukte, die aus dem Herstellungsverfahren abgetrennt und üblicherweise als nicht verwertbarer Rückstand verworfen werden. Das Wertprodukt (Destillat) wird mit der gleich großen oder vorzugsweise größeren anderen Teilmenge vereinigt und die vereinigte Diurethane enthaltende Reaktionsmischung der thermischen Spaltung zugeführt.

Durch diese Verfahrensmaßnahme wird der Anteil an nicht destillierbaren Nebenprodukten in der Reaktionsmischung, die sich bei den nacheinander ablaufenden Teilreaktionen bilden und durch die Rückführung verwertbarer Einsatzstoff im Reaktionskreislauf ständig anreichern würden, auf einen Gehalt von 3 bis 30 Gew.%, vorzugsweise 5 bis 20 Gew.% begrenzt und dadurch eine mit hoher Selektivität störungsfrei ablaufende Reaktion gewährleistet.

Als Destillationseinrichtungen können Dünnschichtverdampfer oder Kurzwegverdampfer zum Einsatz kommen. Das Urethan wird bei Drücken von 0,001 - 1 bar, vorzugsweise im Bereich von 0,002 - 0,01 bar destilliert. Das Destillat wird der Spaltung zugeführt.

Der hochsiederhaltige Sumpf wird bevorzugt verworfen oder kann weniger bevorzugt teilweise der Reurethanisierung zugeführt werden.

Die so erhaltene Diurethane-enthaltende Reaktionsmischung wird in einer geeigneten Vorrichtung, bevorzugt lösungsmittelfrei in flüssiger Phase in Gegenwart von Katalysatoren bei Temperaturen von 200 bis 300°C, vorzugsweise 220 bis 280°C und unter vermindertem Druck von 0,01 - 0,6 bar, vorzugsweise im Bereich von 0,02 - 0,1 bar kontinuierlich thermisch gespalten. Der Umsatz von Diurethan zu Diisocyanat in der Vorrichtung zur thermischen Spaltung kann weitgehend frei gewählt werden und liegt zweckmäßigerweise in einem Bereich von 10 bis 98 Gew.%, vorzugsweise 40 bis 90 Gew.% der zugeführten Menge.

Der ungespaltene Anteil der Reaktionsmischung, der nicht umgesetzte Diurethane, Oligoharnstoff-polyurethane, hochsiedende Oligomere und andere wieder verwertbare und unverwertbare Nebenprodukte enthält, wird abgetrennt, kontinuierlich aus der Spaltvorrichtung ausgeschleust und direkt oder gegebenenfalls nach Umsetzung mit Alkohol in der Reurethanisierung in die Reaktionsstufe zurückgeführt.

Als Katalysatoren zur chemischen Spaltung finden z.B. die vorgenannten, die Urethanbildung katalysierende anorganischen und organischen Verbindungen Verwendung.

Besonders bewährt und daher vorzugsweise verwendet werden Dibutylzinndilaurat, Eisen-(III)-acetylacetonat, Kobalt-(II)-acetylacetonat, Zinkacetylacetonat, Zirkon tetra-n-butanolat und Zinn-(II)-dioctoat.

Als Spaltvorrichtungen eignen sich beispielsweise zylinderförmige Spaltreaktoren, wie z.B. Röhrenöfen oder vorzugsweise Verdampfer, beispielsweise Dünnschicht- oder Bulkverdampfer, wie z.B. Robertverdampfer, Herbertverdampfer, caddle-typ-Verdampfer, Plattenspalter und vorzugsweise Heizkerzenverdampfer.

Die Trennung der Spaltprodukte erfolgt in einer Kolonne, bei der üblicherweise das Isocyanat in der Seite und der Alkohol am Kopf abgezogen werden.

Das Rohisocyanatgemisch wird in einer sich anschließenden Destillation von Rekombinationsprodukten, Nebenprodukten und sofern vorhanden dem Lösungsmittel befreit. Die Nebenprodukte werden vorzugsweise in die thermische Spaltung zurückgeführt. Ein Teil kann auch ausgeschleust werden.

Die bei der thermischen Spaltung gebildeten Spaltprodukte, die sich vor allem aus Alkohol, Diisocyanat, und partiell gespaltenen Diurethanen zusammensetzen, werden danach vorteilhafterweise mit Hilfe einer oder mehrerer Destillationskolonnen, vorzugsweise durch Rektifikation bei Temperaturen von 100 bis 220°C, vorzugsweise 120 bis 170°C und einem Druck von 1 bis 200 mbar, vorzugsweise 5 bis 50 mbar, in Leichtsieder und besonders Alkohol und eine rohe Diisocyanatmischung mit einem Diisocyanatgehalt von 85 bis 99 Gew.%, vorzugsweise von 95 bis 99 Gew.% getrennt. Die bei der destillativen Trennung anfallenden höhersiedenden Nebenprodukte und insbesondere die ungespaltenen und partiell gespaltenen Diurethane werden vorzugsweise in die Spaltvorrichtung und/oder Reurethanisierung geführt.

Die vorzugsweise durch Rektifikation erhaltene rohe Isocyanatmischung wird durch Destillation bei einer Temperatur von 100 bis 180°C und unter einem Druck von 1 bis 50 mbar gereinigt, wobei die einzelnen Fraktionen zurückgeführt oder als Reinprodukt isoliert werden. Wie bereits ausgeführt wurde, wird bei der bevorzugt angewandten Reindestillation die Kopffraktion, die vorzugsweise aus Diisocyanat besteht, gegebenenfalls nach Umsetzung der freien Isocyanatgruppen mit Alkohol in die Reaktionsstufe zurückgeführt, die Seitenfraktion, die aus reinem Diisocyanat, vorzugsweise mit einer Reinheit von mindestens 98 Gew.%, insbesondere über 99 Gew.% besteht, wird abgeleitet und der Lagerung zugeführt und die Sumpffraktion, die als wesentliche Komponenten die partiell gespaltenen Diurethane und Diisocyanate enthält, wird vorzugsweise in die Spaltvorrichtung zur thermischen Spaltung zurückgeführt.

Die Umsetzung des Reaktionsaustrages und/oder Destillationsrückstände werden vorzugsweise erneut dem Prozess zugeführt. Dabei werden mit Alkohol die in diesem Gemisch enthaltenen Isocyanatgruppen und/oder Allophanate und/oder Harnstoffe oder sonstige reaktive Bestandteile zu Urethanen umgewandelt. Es besteht die Möglichkeit, diese Reaktionen in separaten Reaktoren wie z. B. Mischreaktoren oder Strömungsrohren oder auch in durchzuführen. Für die Alkoholyse der Rückstände sind Temperaturen von 100 - 250 °C, vorzugsweise 150 - 220 °C erforderlich. Die mittleren Verweilzeiten liegen dabei im Bereich von wenigen Minuten bis Stunden.

Dazu können beispielsweise die Ströme mit Alkohol zusammengeführt werden, wobei das Molverhältnis von NCO-Gruppen bzw. deren Äquivalenten, also beispielsweise Urethangruppen, zu Hydroxygruppen bis zu 1:100, bevorzugt bis zu 1:20, besonders bevorzugt bis zu 1:10 beträgt.

Diese Reaktionsmischung wird in Gegenwart oder Abwesenheit von Katalysatoren innerhalb von 1 bis 150 min, bevorzugt 3 bis 60 min bei Temperatur von 20 bis 200 °C, bevorzugt 50 bis 170 °C bei einem Druck von 0,5 bis 20 bar, bevorzugt 1 bis 15 bar umgesetzt.

Die Umsetzung kann in einer kontinuierlichen Kesselkaskade oder in einem Rohrreaktor durchgeführt werden.

Als Katalysatoren kommen grundsätzlich alle Verbindungen in Frage, die die Reaktion von NCO- mit OH-Gruppen fördern. Beispielsweise seien genannt Zinnoctoat, Dibutylzinndilaurat, Zinnchlorid, Zinkdichlorid, Zinn-(II)-dioctoat und Triethylamin.

Die Durchführung der Phosgenierung in der Flüssigphase ist ebenfalls an sich bekannt und kann bevorzugt wie folgt ausgeführt werden:

Das aus Schritt b) erhaltene 1,5-Pentandiamin wird wahlweise in freier Form oder als Hydrochlorid gegebenenfalls in einem Lösungsmittel vorgelöst.

Der Wassergehalt des in die Stufe c) eingesetzten 1,5-Pentandiamin richtet sich nach der Art der Reaktion in der Stufe c) und sollte im Falle einer Phosgenierung bevorzugt unter 200 Gew.ppm, im Falle einer phosgenfreien Durchführung bevorzugt unter 10 Gew%, besonders bevorzugt unter 1 Gew% und ganz besonders bevorzugt unter 1000 Gew.ppm betragen.

Dabei sind Chlorbenzol, o- oder p-Dichlorbenzol, Trichlorbenzol, Chlortoluole, Chlorxylole, Chlorethylbenzol, Chlornaphthaline, Chlordiphenyle, Methylenchlorid, Perchlorethylen, Toluol, Xylole, Hexan, Dekahydronaphthalin, Diethylisophthalat (DEIP) und andere Carbonsäureester, wie sie beispielsweise in der US 5,136,086, Spalte 3, Zeilen 3 bis 18 aufgeführt sind, Tetrahydrofuran (THF), Dimethylformamid (DMF), Benzol und deren Gemische bevorzugt. Besonders bevorzugt ist Chlorbenzol und Dichlorbenzol.

Der Gehalt an Amin im Gemisch Amin/Lösungsmittel beträgt üblicherweise zwischen 1 und 50 Massen-%, bevorzugt zwischen 2 und 40 Massen-%, besonders bevorzugt zwischen 3 und 30 Massen%.

Das Phosgen wird als Gemisch mit dem gleichen oder einem anderen inerten Lösungsmittel, bevorzugt dem gleichen, oder rein eingesetzt. Besonders bevorzugt wird als Phosgen zumindest teilweise ein rückgewonnener Strom aus der Aufarbeitung verwendet, der entsprechend der gewünschten Stöchiometrie durch frisches Phosgen ergänzt wird.

Das Phosgen kann beim erfindungsgemäßen Verfahren im allgemeinen in Form von 10- bis 100-, vorzugsweise 30-bis 95- und insbesondere 40- bis 90-gew.-%igen, Lösungen in inerten Lösungsmitteln zum Einsatz kommen, wobei vorzugsweise für das Phosgen das gleiche Lösungsmittel wie für das Amin verwendet wird.

Die Temperatur der Phosgenlösung sollte zwischen -35 °C und 180 °C, bevorzugt zwischen -30 °C und 150°C betragen.

Beispielsweise kann die Temperatur des Aminzulaufs zur Mischeinrichtung zwischen 10 und 150 °C, bevorzugt 15 - 120 °C und besonders bevorzugt 20 - 100 °C betragen.

Das molare Verhältnis von insgesamt in die Reaktion eingespeistem Phosgen zu eingesetzten Aminogruppen beträgt im allgemeinen 1,1 : 1 bis 30 : 1, bevorzugt von 1,3:1 bis 25:1.

Die Vermischung der Eduktströme erfolgt bevorzugt in einer geeigneten speziellen Mischeinrichtung, die sich durch geringe Mischzeiten auszeichnet.

Die mittlere Verweilzeit in der Reaktion nach der Vermischung beträgt in der Regel 5 min bis 15 h, bevorzugt 10 min bis 12 h, besonders bevorzugt 15 min bis 10 h.

Die Temperatur in der Reaktion beträgt im allgemeinen zwischen 90 °C und 250 °C, bevorzugt zwischen 100 °C und 240 °C und besonders bevorzugt zwischen 110 und 230 °C.

Der Druck in der Reaktion beträgt in der Regel zwischen 1,1 bar und 80 bar abs, bevorzugt zwischen 1,5 und 50 bar abs, besonders bevorzugt zwischen 2 und 35 bar abs, ganz besonders bevorzugt zwischen 3 und 10 bar abs, und insbesondere zwischen 4 und 8 bar abs.

Die Reaktion kann in einem rückvermischten Reaktor oder in einem Rohrreaktor erfolgen, oder auch in einer Kombination aus einem rückvermischten Reaktor, dem ein Rohrreaktor nachgeschaltet ist.

Das Reaktionsgemisch wird anschließend destillativ gereinigt.

Beispielsweise kann es sich dabei um eine Destillationskolonne handeln. Diese Destillationseinheit ist von an sich bekannter Bauart und weist die üblichen Einbauten auf. Als Kolonneneinbauten kommen prinzipiell alle gängigen Einbauten in Betracht, beispielsweise Böden, Packungen und/oder Schüttungen. Von den Böden sind Glockenböden, Siebböden, Ventilböden, Thormannböden und/oder Dual-Flow-Böden bevorzugt, von den Schüttungen sind solche mit Ringen, Wendeln, Sattelkörpern, Raschig-, Intos- oder Pall-Ringen, Barrel- oder Intalox-Sätteln, Top-Pak etc. oder Geflechten bevorzugt. Bevorzugt werden Böden verwendet, besonders bevorzugt Glockenböden.

Die Destillationskolonne weist bevorzugt 10 - 80 theoretische Trennböden auf.

In dieser Kolonne werden die Gasphase von unten nach oben und die Flüssigphase von oben nach unten durch die Kolonne geführt.

Die Erzeugung der Gasphase im Sumpf der Kolonne erfolgt durch den Betrieb eines Verdampfers, der in den Sumpf eingebaut sein kann, beispielsweise ein RobertVerdampfer, oder im Umlauf mit einem externen Verdampfer, z. B. Röhren- oder Plattenwärmetauscher.

Ein Umlauf ist dann beispielsweise ein Zwangsumlauf oder ein Naturumlauf. Bevorzugt erfolgt die Verdampfung in einem Naturumlauf.

Eine weitere erfindungsgemäße besteht in der Erzeugung eines Gasstromes in der Kolonne durch Einblasen von gasförmigen oder überhitztem Phosgen und/oder inertem Lösungsmittel und/oder inerten Gasen.

Die mittlere Verweilzeit in der Kolonne beträgt zwischen 10 min und 12 h, bevorzugt 15 min - 11 h und besonders bevorzugt 15 min - 10 h.

Die Sumpftemperatur in der Destillationskolonne beträgt im allgemeinen zwischen 90 °C und 250 °C, bevorzugt zwischen 100 °C und 240 °C und besonders bevorzugt zwischen 110 und 230 °C. Der Kopfdruck in der Destillationskolonne beträgt in der Regel zwischen 1,1 bar abs und 80 bar abs, bevorzugt zwischen 1,5 und 50 bar abs, besonders bevorzugt zwischen 2 und 35 bar abs, ganz besonders bevorzugt zwischen 3 und 10 bar abs und insbesondere zwischen 4 und 8 bar abs.

Am Sumpf der Kolonne wird dann ein das Isocyanat als Produkt enthaltender flüssiger und/oder gasförmiger Strom entnommen.

Die Phosgenierung in der Gasphase kann beispielsweise erfolgen, wie beschrieben in EP 1 275 639 A1, EP 1 275 640 A1, EP 1 449 826 A1, DE 10359627 A1 oder in der deutschen Patentanmeldung DE 102005042392.

Bevorzugt kann die Gasphasenphosgenierung durchgeführt werden wie folgt:

Bei der Gasphasenphosgenierung ist es definitionsgemäß anzustreben, daß die im Reaktionsverlauf auftretenden Verbindungen, also Edukte (Diamin und Phosgen), Zwischenprodukte (insbesondere die intermediär entstehenden Mono- und Dicarbamoychloride), Endprodukte (Diisocyanat), sowie gegebenenfalls zudosierte inerte Verbindungen, unter den Reaktionsbedingungen in der Gasphase verbleiben. Sollten diese oder andere Komponenten sich aus der Gasphase z.B. an der Reaktorwand oder anderen Apparatebauteilen abscheiden, so kann durch diese Abscheidungen der Wärmeübergang oder die Durchströmung der betroffenen Bauteile unerwünscht verändert werden. Dies gilt insbesondere für auftretende Aminhydrochloride, die sich aus freien Aminogruppen und Chlorwasserstoff (HCl) bilden, da die resultierenden Aminhydrochloride leicht ausfallen und nur schwer wieder verdampfbar sind.

Die Edukte, oder auch nur eines von ihnen, können zusammen mit mindestens einem Inertmedium in den Mischraum eindosiert werden.

Bei dem Inertmedium handelt es sich um ein Medium, das bei der Reaktionstemperatur gasförmig im Reaktionsraum vorliegt und nicht mit den im Reaktionsverlauf auftretenden Verbindungen reagiert. Das Inertmedium wird im allgemeinen vor der Umsetzung mit Amin und/oder Phosgen vermischt, kann aber auch getrennt von den Eduktströmen zudosiert werden. Beispielsweise können Stickstoff, Edelgase, wie Helium oder Argon, oder Aromaten, wie Chlorbenzol, Chlortoluol, o-Dichlorbenzol, Toluol, Xylol, Chlornaphthalin, Decahydronaphthalin, Kohlenstoffdioxid oder Kohlenstoffmonoxid, verwendet werden. Bevorzugt wird Stickstoff und/oder Chlorbenzol als Inertmedium verwendet.

Im allgemeinen wird das Inertmedium in einer Menge eingesetzt, so dass das Verhältnis der Gasvolumina von Inertmedium zu Amin bzw. zu Phosgen mehr als 0,0001 bis 30, bevorzugt mehr als 0,01 bis 15, besonders bevorzugt mehr als 0,1 bis 5 beträgt.

Die Ausgangsamine werden vor der Durchführung des erfindungsgemäßen Verfahrens verdampft und auf 200°C bis 600°C, vorzugsweise 300°C bis 500°C erhitzt und gegebenenfalls verdünnt mit einem Inertgas oder mit den Dämpfen eines inerten Lösungsmittels durch die Mischeinrichtung dem Reaktor zugeführt.

Das bei der Phosgenierung verwendete Phosgen wird vor Durchführung des erfindungsgemäßen Verfahrens gegebenenfalls verdünnt mit einem Inertgas oder mit den Dämpfen eines inerten Lösungsmittels ebenfalls auf eine Temperatur innerhalb des Bereichs von 200°C bis 600°C, vorzugsweise 300°C bis 500°C erhitzt. Erfindungsgemäß wird Phosgen im Überschuss bezüglich Aminogruppen eingesetzt. Üblicherweise liegt ein molares Verhältnis von Phosgen zu Aminogruppen von 1,1 :1 bis 20 : 1, bevorzugt von 1,2 :1 bis 5 :1 vor.

Die Reaktion setzt in der Regel mit Kontakt der Edukte unmittelbar nach der Vermischung ein.

In der Mischeinrichtung werden die Eduktströme in kurzer Zeit möglichst vollständig vermischt.

Zur Durchführung der erfindungsgemäßen Umsetzung werden der vorerhitzte Strom enthaltend Amin oder Gemische von Aminen und der vorerhitzte Strom enthaltend Phosgen kontinuierlich in den Reaktor, bevorzugt einen Rohrreaktor geleitet.

Die Reaktoren bestehen im allgemeinen aus Stahl, Glas, legiertem oder emaillierten Stahl und weisen eine Länge auf, die ausreichend ist, um unter den Verfahrensbedingungen eine vollständige Umsetzung des Diamins mit dem Phosgen zu ermöglichen.

Es können im allgemeinen die aus dem Stand der Technik bekannten Reaktorbautypen verwendet werden. Beispiele für Reaktoren sind bekannt aus EP-B1 289840, Sp. 3, Z. 49 - Sp. 4, Z. 25, EP-B1 593334, WO 2004/026813, S. 3, Z. 24 - S. 6, Z. 10, WO 03/045900, S. 3, Z. 34 - S. 6, Z. 15, EP-A1 1275639, Sp. 4, Z. 17 - Sp. 5, Z. 17 und EP-B1 570799, Sp. 2, Z. 1 - Sp. 3, Z. 42, auf die jeweils im Umfang dieser Offenbarung ausdrücklich Bezug genommen sei.

Bevorzugt verwendet werden Rohrreaktoren.

Die Umsetzung von Phosgen mit Amin im Reaktionsraum erfolgt bei Absolutdrücken von mehr als 0,1 bar bis weniger als 20 bar, bevorzugt zwischen 0,5 bar und 15 bar und besonders bevorzugt zwischen 0,7 und 10 bar. Im Fall der Umsetzung von (cyclo)aliphatischen Aminen beträgt der Absolutdruck ganz besonders bevorzugt zwischen 0,7 bar und 5 bar, insbesondere von 0,8 bis 3 bar und speziell 1 bis 2 bar.

Im allgemeinen ist der Druck in den Zuleitungen zur Mischvorrichtung höher, als der vorstehend angegebene Druck im Reaktor. Je nach Wahl der Mischvorrichtung fällt an dieser Druck ab. Bevorzugt ist der Druck in den Zuleitungen um 20 bis 2000 mbar, besonders bevorzugt von 30 bis 1000 mbar höher als im Reaktionsraum.

Gemäß dem erfindungsgemäßen Verfahren erfolgt die Umsetzung von Phosgen mit Amin in der Gasphase. Unter Umsetzung in der Gasphase ist zu verstehen, dass die Umwandlung der Eduktströme und Zwischenprodukte zu den Produkten im gasförmigen Zustand miteinander reagieren und im Verlauf der Reaktion während des Durchgangs durch den Reaktionsraum zu mindestens 95%, bevorzugt zu mindestens 98%, besonders bevorzugt zu mindestens 99%, ganz besonders bevorzugt zu mindestens 99,5%, insbesondere zu mindestens 99,8 und speziell zu mindestens 99,9% in der Gasphase bleiben.

Zwischenprodukte sind dabei beispielsweise die aus den Diaminen gebildeten Monoamino-monocarbamoylchloride, Dicarbamoylchloride, Monoamino-monoisocyanate und Monoisocyanato-monocarbamoylchloride sowie die Hydrochloride der Aminoverbindungen.

Bei dem erfindungsgemäßen Verfahren wird die Temperatur im Reaktionsraum so gewählt, dass sie oberhalb der Siedetemperatur des eingesetzten Diamins, bezogen auf die im Reaktionsraum herrschenden Druckverhältnisse, liegt. Je nach eingesetztem Amin und eingestelltem Druck ergibt sich üblicherweise eine vorteilhafte Temperatur im Reaktionsraum von mehr als 200 °C, bevorzugt mehr als 260 °C und besonders bevorzugt mehr als 300 °C. In der Regel beträgt die Temperatur bis zu 600, bevorzugt bis zu 570 °C.

Die mittlere Kontaktzeit des Umsetzungsgemisches im erfindungsgemäßen Verfahren beträgt im allgemeinen zwischen 0,001 Sekunden und weniger als 5 Sekunden, bevorzugt von mehr als 0,01 Sekunden bis weniger als 3 Sekunden, besonders bevorzugt von mehr als 0,015 Sekunden bis weniger als 2 Sekunden. Die mittlere Kontaktzeit ganz besonders bevorzugt von 0,015 bis 1,5 Sekunden, insbesondere von 0,015 bis 0,5 Sekunden, speziell von 0,020 bis 0,1 Sekunden und oft von 0,025 bis 0,05 Sekunden betragen.

Bevorzugt durchläuft das gasförmige Reaktionsgemisch den Reaktionsraum mit einer Strömungsgeschwindigkeit von 10 bis 300 Meter/Sekunde, bevorzugt von 25 bis 250 Meter/Sekunde, besonders bevorzugt 40 bis 230, ganz besonders bevorzugt 50 bis 200, insbesondere mehr als 150 bis 190 und speziell 160 bis 180 Meter/Sekunde.

Durch die turbulente Strömung werden enge Verweilzeiten mit geringer Standardabweichung von meist nicht mehr als 6% wie in EP 570799 beschrieben und eine gute Vermischung erreicht. Maßnahmen, wie beispielsweise die in EP-A-593 334 beschriebene Verengung, die zudem verstopfungsanfällig ist, sind nicht notwendig.

Nach der Reaktion wird das gasförmige Umsetzungsgemisch bevorzugt bei Temperaturen größer 130 °C mit einem Lösungsmittel gewaschen (Quench). Als Lösungsmittel sind bevorzugt Kohlenwasserstoffe, die gegebenenfalls mit Halogenatomen substituiert sind, geeignet, wie beispielsweise Hexan, Benzol, Nitrobenzol, Anisol, Chlorbenzol, Chlortoluol, o-Dichlorbenzol, Trichlorbenzol, Diethylisophthalat (DEIP), Tetrahydrofuran (THF), Dimethylformamid (DMF), Xylol, Chlornaphthalin, Decahydronaphthalin und Toluol. Als Lösungsmittel wird besonders bevorzugt Monochlorbenzol eingesetzt. Als Lösungsmittel kann auch das Isocyanat eingesetzt werden. Bei der Wäsche wird das Isocyanat selektiv in die Waschlösung übergeführt. Anschließend werden das verbleibende Gas und die erhaltene Waschlösung bevorzugt mittels Rektifikation in Isocyanat, Lösungsmittel, Phosgen und Chlorwasserstoff aufgetrennt.

Nachdem das Reaktionsgemisch im Reaktionsraum umgesetzt wurde, führt man es in die Aufarbeitungsvorrichtung mit Quench. Bevorzugt handelt es sich hier um einen sogenannten Waschturm, wobei aus dem gasförmigen Gemisch das gebildete Isocyanat durch Kondensation in einem inerten Lösungsmittel abgetrennt wird, während überschüssiges Phosgen, Chlorwasserstoff und gegebenenfalls das Inertmedium die Aufarbeitungsvorrichtung gasförmig durchlaufen. Bevorzugt wird dabei die Temperatur des inerten Lösungsmittels oberhalb der Lösungstemperatur des zum Amin gehörigen Carbamylchlorids im gewählten Quenchmedium gehalten. Besonders bevorzugt wird dabei die Temperatur des inerten Lösungsmittels oberhalb der Schmelztemperatur des zum Amin gehörigen Carbamylchlorids gehalten

Im allgemeinen ist der Druck in der Aufarbeitungsvorrichtung niedriger als im Reaktionsraum. Bevorzugt ist der Druck um 50 bis 500 mbar, besonders bevorzugt 80 bis 150 mbar, niedriger als im Reaktionsraum.

Die Wäsche kann beispielsweise in einem Rührbehälter oder in anderen herkömmlichen Apparaturen, z.B. in einer Kolonne oder Mixer-Settler-Apparatur, durchgeführt werden.

Verfahrenstechnisch können für eine Wäsche im erfindungsgemäßen Verfahren alle an sich bekannten Extraktions- und Waschverfahren und -apparate eingesetzt werden, z.B. solche, die in Ullmann's Encyclopedia of Industrial Chemistry, 6th ed, 1999 Electronic Release, Kapitel: Liquid - Liquid Extraction - Apparatus, beschrieben sind. Beispielsweise können dies ein- oder mehrstufige, bevorzugt einstufige Extraktionen, sowie solche in Gleich- oder Gegenstromfahrweise, bevorzugt Gegenstromfahrweise sein.

Ein geeigneter Quench ist beispielsweise bekannt aus EP-A1 1403248, Sp. 2, Z. 39 - Sp. 3, Z. 18, auf die im Umfang dieser Offenbarung ausdrücklich Bezug genommen sei.

In dieser Quenchzone wird das Reaktionsgemisch, das im wesentlichen aus den Isocyanaten, Phosgen und Chlorwasserstoff besteht, intensiv mit der eingedüsten Flüssigkeit vermischt. Die Vermischung erfolgt derart, dass die Temperatur des Reaktionsgemisches ausgehend von 200 bis 570°C auf 100 bis 200 °C, bevorzugt auf 140 bis 180 °C abgesenkt wird und das im Reaktionsgemisch enthaltene Isocyanat durch Kondensation vollständig oder teilweise in die eingedüsten Flüssigkeitströpfchen übergeht, während das Phosgen und der Chlorwasserstoff im wesentlichen vollständig in der Gasphase verbleiben.

Der Anteil des im gasförmigen Reaktionsgemisch enthaltenen Isocyanats, das in der Quenchzone in die Flüssigphase übergeht, beträgt dabei vorzugsweise 20 bis 100 Gew.-%, besonders bevorzugt 50 bis 99,5 Gew.-% und insbesondere 70 bis 99 Gew.-%, bezogen auf das im Reaktionsgemisch enthaltene Isocyanat.

Das Reaktionsgemisch durchströmt die Quenchzone vorzugsweise von oben nach unten. Unterhalb der Quenchzone ist ein Sammelbehälter angeordnet, in dem die Flüssigphase abgeschieden, gesammelt und, über einen Auslass aus dem Reaktionsraum entfernt und anschließend aufgearbeitet wird. Die verbleibende Gasphase wird über einen zweiten Auslass aus dem Reaktionsraum entfernt und ebenfalls aufgearbeitet.

Der Quench kann beispielsweise erfolgen, wie in der EP 1403248 A1 beschrieben, oder wie in der internationalen Anmeldung WO 2005/123665 beschrieben.

Die Flüssigkeitströpfchen werden dazu mittels Ein- oder Zweistoffzerstäuberdüsen, vorzugsweise Einstoffzerstäuberdüsen, erzeugt und erzeugen je nach Ausführungsform einen Sprühkegelwinkel von 10 bis 140°, bevorzugt von 10 bis 120°, besonders bevorzugt von 10° bis 100°.

Die Flüssigkeit, die über die Zerstäuberdüsen eingedüst wird, muss eine gute Löslichkeit für Isocyanate aufweisen. Vorzugsweise werden organische Lösungsmittel eingesetzt. Insbesondere eingesetzt werden aromatische Lösungsmittel, die mit Halogenatomen substituiert sein können.

Die Aufarbeitung des so erhaltenen Diisocyanats kann in an sich bekannter Weise erfolgen, beispielsweise wie oben bei der Flüssigphasenphosgenierung beschrieben.

Ein weiterer Gegenstand der vorliegenden Erfindung ist 1,5-Pentamethylendiisocyanat mit einem ¹⁴C : ¹²C-Isotopenverhältnis von 0,5 × 10⁻² bis 5 × 10⁻¹², bevorzugt 1,0 × 10⁻¹² bis 4 x 10⁻¹² , und besonders bevorzugt 1,5 × 10⁻¹² bis 3 × 10⁻¹². Derartiges 1,5-Pentamethylendiisocyanat ist erhältlich, wenn man den Schritt a) bzw. b) ausgehend von biologischem Material durchführt.

Vorteil eines solchen 1,5-Pentamethylendiisocyanats ist, daß es einen ¹⁴C-Isotopengehalt aufweist, der natürlichen Material entspricht, wohingegen 1,5-Pentamethylendiisocyanat, das auf petrochemischer Basis hergestellt wird, einen unnatürlichen Gehalt aufweist, der in der Regel unter 0,3 x 10⁻¹² beträgt, meist unter 0,2 × 10⁻¹² und meist unter 0,1 × 10⁻¹². Dieses erfindungsgemäße 1,5-Pentamethylendiisocyanat kann dann wegen seines Isotopengehalts zur Synthese von Verbindungen eingesetzt werden, die als Sonden für z.B. ¹⁴C-Untersuchungen eingesetzt werden sollen.

Ein weiterer Gegenstand der vorliegenden Erfindung ist 1,5-Pentamethylendiisocyanat, das zusätzlich einen Gesamtchlorgehalt unter 50 Gew.ppm und einen Gehalt an hydrolysierbarem Chlor unter 10 Gew.ppm aufweist. Derartiges 1,5-Pentamethylendiisocyanat ist erhältlich, wenn man den Schritt c) phosgenfrei durchführt. Auf diese Weise ist 1,5-Pentamethylendiisocyanat erhältlich, das vollständig unter Verzicht auf Petro- und Chlorchemie hergestellt worden ist.

Das erfindungsgemäß hergestellte 1,5-Pentamethylendiisocyanat eignet sich durch seine oben angeführten vorteilhaften Eigenschaften vorzüglich zur Herstellung von Isocyanuratgruppen aufweisenden Polyisocyanaten, Uretdiongruppen aufweisenden Polyisocyanaten, Biuretgruppen aufweisenden Polyisocyanaten, Urethan- oder Allophanatgruppen aufweisenden Polyisocyanaten, Oxadiazintriongruppen oder Iminooxadiazindiongruppen enthaltenden Polyisocyanaten und/oder Uretonimin-modifizierten Polyisocyanaten.

Derartige Polyisocyanate finden beispielsweise Anwendung in der Herstellung von Urethan-, Isocyanurat-, Amid- und/oder Harnstoffgruppen enthaltenden Kunststoffen nach dem Polyisocyanat-Polyadditionsverfahren. Derartige Polyisocyanatmischungen werden insbesondere zur Herstellung von lichtbeständigen Polyurethanlacken und -überzügen verwendet.

Die so erhältlichen Polyisocyanate auf Basis des erfindungsgemäß hergestellten 1,5-Pentamethylendiisocyanats werden in der Regel in der Lackindustrie verwendet. Die die erfindungsgemäßen Gemische können beispielsweise in Beschichtungsmitteln für 1 K- oder 2K-Polyurethanlacke eingesetzt werden, beispielsweise für Grundierungen, Füller, Basecoats, unpigmentierte Decklacke, pigmentierte Decklacke und Klarlacke im Bereich Industrie-, insbesondere Flugzeug- oder Großfahrzeuglackierung, Holz-, Auto-, insbesondere OEM- oder Autoreparaturlackierung, oder Dekolackierung eingesetzt werden. Besonders geeignet sind die Beschichtungsmittel für Anwendungen, in denen eine besonders hohe Applikationssicherheit, Außenwitterungsbeständigkeit, Optik, Lösemittel- und/oder Chemikalienfestigkeit gefordert werden. Die Härtung dieser Beschichtungsmittel ist dabei erfindungsgemäß nicht wesentlich. Insbesondere in der Automobilindustrie werden zunehmend Mehrschichthärtungen, z.B. von Clear- und Basecoat, (sogenanntes two-in one), oder von Füller, Clear- und Basecoat, (sogenanntes three-in-one), durchgeführt.

Ferner kann das erfindungsgemäß hergestellte 1,5-Pentamethylendiisocyanat zur Herstellung von thermoplastischen Polyurethanen (TPU) eingesetzt werden, wie es beispielsweise im Kunststoffhandbuch, Band 7 "Polyurethane", Carl Hanser Verlag München Wien, 3. Auflage 1993, Seiten 455 bis 466 beschrieben ist.

Ihre Herstellung erfolgt durch Umsetzung von Diisocyanaten mit Verbindungen mit mindestens zwei mit Isocyanatgruppen reaktiven Wasserstoffatomen, vorzugsweise difunktionellen Alkoholen.

Als gegenüber Isocyanaten reaktive Verbindungen können allgemein bekannte Polyhydroxylverbindungen mit Molekulargewichten von 500 bis 8000, bevorzugt 600 bis 6000, insbesondere 800 bis 4000, und bevorzugt einer mittleren Funktionalität von 1,8 bis 2,6, bevorzugt 1,9 bis 2,2, insbesondere 2 eingesetzt werden, beispielsweise Polyesterole, Polyetherole und/oder Polycarbonatdiole. Bevorzugt werden Polyesterdiole eingesetzt, die erhältlich sind durch Umsetzung von Butandiol und Hexandiol als Diol mit Adipinsäure als Dicarbonsäure, wobei das Gewichtsverhältnis von Butandiol zu Hexandiol bevorzugt 2 zu 1 beträgt. Bevorzugt ist weiterhin Polytetrahydrofuran mit einem Molekulargewicht von 750 bis 2500 g/mol, bevorzugt 750 bis 1200 g/mol.

Als Kettenverlängerungsmittel können allgemein bekannte Verbindungen eingesetzt werden, beispielsweise Diamine und/oder Alkandiole mit 2 bis 10 C-Atomen im Alkylenrest, insbesondere Ethylenglykol und/oder Butandiol-1,4, und/oder Hexandiol und/oder Di- und/oder Tri-oxyalkylenglykole mit 3 bis 8 Kohlenstoffatomen im Oxyalkylenrest, bevorzugt entsprechende Oligo-Polyoxypropylenglykole, wobei auch Mischungen der Kettenverlängerer eingesetzt werden können. Als Kettenverlängerer können auch 1,4-Bis-(hydroxymethyl)-benzol (1,4-BHMB), 1,4-Bis-(hydroxyethyl)-benzol (1,4-BHEB) oder 1,4-Bis-(2-hydroxyethoxy)-benzol (1,4-HQEE) zum Einsatz kommen. Bevorzugt werden als Kettenverlängerer Ethylenglykol und Hexandiol, besonders bevorzugt Ethylenglykol.

Üblicherweise werden Katalysatoren eingesetzt, welche die Reaktion zwischen den NCO-Gruppen der Diisocyanate und den Hydroxylgruppen der Aufbaukomponenten beschleunigen, beispielsweise tertiäre Amine, wie Triethylamin, Dimethylcyclohexylamin, N-Methylmorpholin, N,N'-Dimethylpiperazin, 2-(Dimethylaminoethoxy)-ethanol, Diazabicyclo-(2,2,2)-octan und ähnliche sowie insbesondere organische Metallverbindungen wie Titansäureester, Eisenverbindungen wie z.B. Eisen-(III)- acetylacetonat, Zinnverbindungen, wie Zinndiacetat, Zinndilaurat oder die Zinndialkylsalze aliphatischer Carbonsäuren wie Dibutylzinndiacetat, Dibutylzinndilaurat oder ähnliche. Die Katalysatoren werden üblicherweise in Mengen von 0,0001 bis 0,1 Gew.-Teilen pro 100 Gew.-Teile Polyhydroxylverbindung eingesetzt.

Neben Katalysatoren können den Aufbaukomponenten bis auch übliche Hilfsstoffe hinzugefügt werden. Genannt seien beispielsweise oberflächenaktive Substanzen, Flammschutzmittel, Keimbildungsmittel, Gleit- und Entformungshilfen, Farbstoffe und Pigmente, Inhibitoren, Stabilisatoren gegen Hydrolyse, Licht, Hitze, Oxidation oder Verfärbung, Schutzmittel gegen mikrobiellen Abbau, anorganische und/oder organische Füllstoffe, Verstärkungsmittel und Weichmacher.

Die Herstellung der TPU erfolgt zumeist nach üblichen Verfahren, wie mittels Bandanlagen oder Reaktionsextruder.

Zur Herstellung von expandierten TPU werden die TPU vorzugsweise mit expandierbaren Mikrospheren gemischt und thermoplastisch zu den gewünschten Formkörpern verarbeitet. Dies kann beispielsweise mittels Spritzguss Sintern oder mittels Extrusion erfolgen. Durch die Temperatur bei der thermoplastischen Verarbeitung kommt es zu einer Expansion der expandierbaren Mikrospheren und somit zur Ausbildung der expandierten TPU. Vorzugsweise wird die Schmelze in Formen eingetragen und härtet dort aus.

Expandierte TPU können beispielsweise als Folien, Schläuche, Profile, Fasern, Kabel, Schuhsohlen, sonstige Schuhteile, Ohrmarken, Automobilteile, Landwirtschaftliche Produkte, Elektroprodukte, Dämpfungselemente; Armlehnen; Kunststoffmöbelelemente, Skischuhe, Anschlagpuffer, Rollen, Skibrillen, Powderslushoberflächen verwendet werden.

Es stellt einen Vorteil des erfindungsgemäßen Verfahrens dar, daß es erstmals eine großtechnische Herstellung von 1,5-Pentandiisocyanat zuläßt. Unter den Begriff "großtechnisch" wird in dieser Schrift die Herstellung von mindestens 50 Tonnen/Jahr, bevorzugt mindestens 500 Tonnen/Jahr und besonders bevorzugt mindestens 1000 Tonnen/Jahr verstanden.

## Patentansprüche

1. 1,5-Pentamethylendiisocyanat mit einem ¹⁴C : ¹²C-Isotopenverhältnis von 0,5 × 10⁻¹² bis 5 × 10⁻¹².

2. Pentamethylen-1,5-diisocyanat gemäß Anspruch 1, **dadurch gekennzeichnet, daß** es eine Farbzahl von nicht mehr als 15 APHA gemäß gemäß DIN ISO 6271 aufweist.

3. Pentamethylen-1,5-diisocyanat gemäß einem der Ansprüche 1 oder 2, **dadurch gekennzeichnet, daß** es einen Anteil an Pentamethylen-1,4-diisocyanat von nicht mehr als 10000 ppm aufweist.

4. 1,5-Pentamethylendiisocyanat gemäß einemd er Ansprüche 1 bis 3 mit einem Gesamtchlorgehalt unter 50 Gew.ppm und einem Gehalt an hydrolysierbarem Chlor unter 10 Gew.ppm.

5. Verwendung von Pentamethylen-1,5-diisocyanat gemäß einem der Ansprüche 2 bis 4 zur Herstellung von Polyisocyanaten oder thermoplastischen Polyurethanen.

6. Verwendung von 1,5-Pentamethylendiisocyanat gemäß einem der vorstehenden Ansprüche zur Herstellung von Isocyanuratgruppen aufweisenden Polyisocyanaten, Uretdiongruppen aufweisenden Polyisocyanaten, Biuretgruppen aufweisenden Polyisocyanaten, Urethan- oder Allophanatgruppen aufweisenden Polyisocyanaten, Oxadiazintriongruppen oder Iminooxadiazindiongruppen enthaltenden Polyisocyanaten und/oder Uretonimin-modifizierten Polyisocyanaten.

7. Polyisocyanate, aufweisend Isocyanuratgruppen, Uretdiongruppen, Biuretgruppen, Urethan- oder Allophanatgruppen, Oxadiazintriongruppen oder Iminooxadiazindiongruppen und/oder Uretonimin-modifizierten Polyisocyanaten hergestellt aus 1,5-Pentamethylendiisocyanat gemäß einem der Ansprüche 1 bis 5.

8. Verwendung von Polyisocyanaten gemäß Anspruch 7 zur Herstellung von Urethan-, Isocyanurat-, Amid- und/oder Harnstoffgruppen enthaltenden Kunststoffen nach dem Polyisocyanat-Polyadditionsverfahren.

9. Verwendung von Polyisocyanaten gemäß Anspruch 7 zur Herstellung von Polyurethanlacken und -überzügen.
